# EUROPÄISCHE PATENTANMELDUNG

(11) **EP 1 256 560 A2**
(43) Veröffentlichungstag der Anmeldung: **13.11.2002**
(21) Anmeldenummer: 02007807.7
(22) Anmeldetag: 06.04.2002
(51) Int. Cl.: C07C 29/42, C07C 33/048, C07C 29/17, C07C 33/02, C07C 45/48, C07C 49/203

(54) **Verfahren zur Herstellung von höheren, alpha,beta-ungesättigten Alkoholen**

(30) Priorität: 11.05.2001 DE 10123066
(71) Anmelder: BASF AKTIENGESELLSCHAFT, 67056 Ludwigshafen (DE)
(72) Erfinder: Ansmann, Andreas, Dr., 69168 Wiesloch (DE); Henkelmann, Jochem, Dr., 68165 Mannheim (DE); Kindler, Alois, Dr., 67269 Grünstadt (DE); Etzrodt, Heinz, Dr., 67434 Neustadt (DE); Oost, Carsten, Dr., 67098 Bad Dürkheim (DE); Stutz, Susanne, Dr., 69469 Weinheim (DE); Tragut, Christian, 2930 Brasschaat (BE); Bockstiegel, Bernhard, Dr., 67354 Römerberg (DE); Reimer, Klaus, 67112 Mutterstadt (DE); Stroezel, Manfred, 68549 Ilvesheim (DE); Dobler, Walter, Dr., 68723 Schwetzingen (DE)

(57) **Zusammenfassung**

Die vorliegende Erfindung betrifft ein Verfahren zur Herstellung von höheren, α,β-ungesättigten Alkoholen durch Monoethinylierung eines Ketons nach dem NH₃/KOH-Verfahren, gegebenenfalls Hydrierung des Acetylenalkohols in Gegenwart von Wasserstoff an einem Pd-haltigen Dünnschichtkatalysator, Reindestillation des Hydrieraustrages vorzugsweise in einer Trennwandkolonne unter Rückführung nicht umgesetzten Ketons in die Ethinylierungsstufe, sowie gegebenenfalls die Herstellung von um jeweils 5 C-Atome längerkettigen höheren Alkoholen, indem die durch Monoethinylierung und gegebenenfalls Partialhydrierung hergestellten Alkohole anschließend mit Acetessigsäurealkylestern oder Diketen in einer Carroll-Reaktion zu Ketonen umgesetzt und diese in die Stufen Ethinylierung, gegebenenfalls Hydrierung und destillative Trennung eingesetzt werden.

## Beschreibung

Die vorliegende Erfindung betrifft ein Verfahren zur Herstellung von höheren, α,β-ungesättigten Alkoholen durch Monoethinylierung eines Ketons nach dem NH₃/KOH-Verfahren, gegebenenfalls Hydrierung des Acetylenalkohols in Gegenwart von Wasserstoff an einem Pd-haltigen Dünnschichtkatalysator, Reindestillation des Hydrieraustrages vorzugsweise in einer Trennwandkolonne unter Rückführung nicht umgesetzten Ketons in die Ethinylierungsstufe, sowie gegebenenfalls die Herstellung von um jeweils 5 C-Atome längerkettigen höheren Alkoholen, indem die durch Monoethinylierung und gegebenenfalls Partialhydrierung hergestellten Alkohole anschließend mit Acetessigsäurealkylestern oder Diketen in einer Carroll-Reaktion zu Ketonen umgesetzt und diese in die Stufen Ethinylierung, gegebenenfalls Hydrierung und destillative Trennung eingesetzt werden.

Die kontinuierlich betriebene Ethinylierung von Ketonen mit Acetylen in flüssigem Ammoniak mit katalytischen Mengen Base (meist KOH oder K-Methylat in einem polaren, protischen Solvens; 10 bis 40°C; 20 bar), wie z.B. beschrieben in DE 1232573, ist Stand der Technik.

Mit dem NH₃/KOH-Verfahren wird in der Regel selektiv der Mono-Acetylenalkohol erhalten. Die Rückführung vom Lösungsmittel NH₃ und von unumgesetztem Acetylen sind ebenfalls Stand der Technik. Sie sind für die Wirtschaftlichkeit der Stufe unabdingbar.

Die Aufarbeitung der Reaktionsausträge durch Neutralisation mit Wasser/CO₂ und anschließende Phasentrennung/Trocknung ist ebenfalls gängiger Stand der Technik.

Die kontinuierlich betriebene Hydrierung von Alkinen und Alkinenen an getränkten Dünnschichtkatalysatoren auf Basis von Pd/Ag sowie die Herstellung dieser Katalysatoren wird in EP 827 944 beschrieben. Bezüglich der Hydrierung mit anderen Katalysatoren oder Verfahren sei auf den in EP 827 944 offenbarten Stand der Technik verwiesen.

Für die kontinuierliche destillative Zerlegung von Mehrstoffgemischen sind verschiedene Verfahrensvarianten gebräuchlich. Im einfachsten Fall wird das Zulaufgemisch in 2 Fraktionen, eine leichtsiedende Kopffraktion und eine schwersiedende Sumpffraktion, zerlegt. Bei der Auftrennung von Zulaufgemischen in mehr als 2 Fraktionen müssen nach dieser Verfahrensvariante mehrere Destillationskolonnen eingesetzt werden. Um den apparativen Aufwand zu begrenzen, setzt man bei der Auftrennung von Vielstoffgemischen nach Möglichkeit Kolonnen mit flüssigen oder dampfförmigen Seitenabzügen ein. Die Anwendungsmöglichkeit von Destillationskolonnen mit Seitenabzügen ist jedoch dadurch stark eingeschränkt, daß die an den Seitenabzugsstellen entnommenen Produkte nie völlig rein sind. Bei Seitenentnahmen im Verstärkungsteil, die üblicherweise in flüssiger Form erfolgen, enthält das Seitenprodukt noch Anteile an leichtsiedenden Komponenten, die über Kopf abgetrennt werden sollen. Entsprechendes gilt für Seitenentnahmen im Abtriebsteil, die meist dampfförmig erfolgen, bei denen das Seitenprodukt noch Hochsiederanteile aufweist. Die Verwendung von konventionellen Seitenabzugskolonnen ist daher auf Fälle begrenzt, in denen verunreinigte Seitenprodukte zulässig sind.

Eine Abhilfemöglichkeit bieten Trennwandkolonnen. Dieser Kolonnentyp ist beispielsweise beschrieben in U.S. 2,471,134; U.S. 4,230,533; EP 0 122 367; EP 0 126 288 und EP 0 133 510.

Bei Trennwandkolonnen ist es im Gegensatz zu Seitenabzugskolonnen möglich, Seitenprodukte ebenfalls in reiner Form zu entnehmen. Im mittleren Bereich oberhalb und unterhalb der Zulaufstelle und der Seitenentnahme ist eine Trennwand angebracht, die den Zulaufteil gegenüber dem Entnahmeteil abdichtet und in diesem Kolonnenteil eine Quervermischung von Flüssigkeits- und Brüdenströmen unterbindet. Hierdurch verringert sich bei der Auftrennung von Vielstoffgemischen die Zahl der insgesamt benötigten Destillationskolonnen. Da dieser Kolonnentyp eine apparative Vereinfachung von thermisch gekoppelten Destillationskolonnen darstellt, weist er darüber hinaus auch einen besonders niedrigen Energieverbrauch auf. Eine Beschreibung von thermisch gekoppelten Destillationskolonnen, die in verschiedener apparativer Ausgestaltung ausgeführt sein können findet sich ebenfalls in den oben genannten Stellen in der Fachliteratur. Trennwandkolonnen und thermisch gekoppelte Kolonnen bieten gegenüber der Anordnung von konventionellen Destillationskolonnen sowohl hinsichtlich des Energiebedarfs als auch der Investitionskosten Vorteile und werden daher zunehmend industriell eingesetzt.

Für die Regelung von Trennwandkolonnen und thermisch gekoppelten Kolonnen werden verschiedene Regelungsstrategien beschrieben. Beschreibungen finden sich in U.S. 4,230,533; DE 35 22 234 und EP-780 147.

Die bevorzugten Varianten zur Durchführung der Carroll-Reaktionen sind in EP 1000 922, EP 1008 582 und EP 0983 988 beschrieben.

Die Herstellung von ungesättigten Ketonen durch Umsetzung von α,β-ungesättigten Alkoholen mit Acetessigsäurealkylestern in Gegenwart von organischen Aluminiumverbindungen unter Abspaltung des aus dem Acetessigester stammenden Alkohols ist in ihren wesentlichen Merkmalen bereits bekannt. Die unkatalysierte Umsetzung zwischen einem ungesättigten Alkohol und einem Acetessigsäurealkylester wurde erstmals von M.F. Carroll [J. Chem. Soc. (London) 1940, S. 704-706] beschrieben. Über den Anwendungsbereich und den Mechanismus dieser Reaktion wurde 1941 von demselben Autor [J. Chem. Soc. (London) 1941, S. 507-511] berichtet.

Eine Verfahrensvorschrift für die Herstellung von 6,10,14-Trimethylpentadec-5-en-2-on durch Umesterung von Acetessigsäureethylester mit 3,7,11-Trimethyl-dodec-l-en-3-ol in Anwesenheit von Aluminiumtrialkoholaten ist der französischen Patentschrift 1 219 166 (1959) zu entnehmen. Gemäß diesem Verfahren werden die Reaktanden und der Katalysator gemeinsam in der Reaktionsblase vorgelegt und die Reaktion unter destillativer Abtrennung des freiwerdenden Alkohols diskontinuierlich durchgeführt. Dabei wird das gewünschte Keton in einer Reaktionszeit von etwa 10 Stunden in 77%iger Ausbeute erhalten. Für eine technische Synthese unbefriedigend sind sowohl die verhältnismäßig langen Reaktionszeiten als auch die unzureichenden Ausbeuten.

Es ist eine Reihe weiterer Patentschriften bekannt, in denen verschiedene Varianten dieser sogenannten Carroll'schen Reaktion beschrieben werden. So heißt es in der US-PS 2 795 617 (1957) bzw. DE-AS 1 053 498 (1959) bzw. CH-PS 342947(1959), daß 'obschon es in der Regel weder notwendig noch erwünscht sei, ein Lösungsmittel verwendet werden kann, um den exothermen Reaktionsverlauf zu mildern'. Nach diesen Patentschriften wird das Aluminiumtrialkoholat zu dem Acetoacetat des α,β-ungesättigten Alkohols gegeben und die Mischung bei starkem Rühren unter Rückfluß erhitzt, wobei Ausbeuten bis zu 80 % erreicht werden. Die Herstellung des entsprechenden Acetoacetats muß in einer vorangehenden Stufe erfolgen.

In der US-PS 2 839 579 (1958) bzw. DE-PS 1078112 (1960) wird berichtet, daß die Umsetzung in einem Lösungsmittel durchgeführt werden kann. Die Herstellung des entsprechenden Acetoacetats erfolgt durch Kondensation von Diketen mit einem entsprechenden ungesättigten Alkohol in einer separaten Stufe. Auch in der DE-PS 1 068 696 heißt es, daß die Mitverwendung eines Lösungsmittels vorteilhaft sein könnte.

In allen Fällen werden hochsiedende Lösungsmittel genannt, deren Siedepunkte weit oberhalb der Reaktionstemperatur liegen. Die in diesen Patenten angegebenen Ausbeuten sind unbefriedigend für eine technische Anwendung. Auch die Mitverwendung eines hochsiedenden Lösungsmittels bringt im allgemeinen keine nennenswerten Ausbeutesteigerungen mit sich und führt daher zu einer Reduzierung der Raum-Zeit-Ausbeuten. Von erheblichem Nachteil ist es, daß zur Herstellung des Acetoacetats des α,β-ungesättigten Alkohols eine weitere Verfahrensstufe erforderlich ist, da hiermit weitere Kosten verbunden sind.

Ein Verfahren zur Herstellung von 2-Methyl-2-hepten-6-on wird in der AS 2652863 (1978) beschrieben. Hierbei werden Acetessigsäurealkylester, Methylbutenol sowie Katalysator in einem Reaktionsgefäß mit aufgesetzter Fraktionierkolonne vorgelegt und anschließend ein Gemisch aus Acetessigsäurealkylester und Methylbutenol zudosiert. Während der Reaktion soll der Gehalt an Acetessigsäurealkylester im Reaktionsgemisch nicht mehr als 15 Gew.-% betragen, um Nebenreaktionen zu vermeiden. Ein Nachteil dieses Verfahren ist jedoch, daß ein einfaches Eindosieren von Acetessigsäurealkylester in überschüssiges Methylbutenol nicht möglich ist, da der Siedepunkt von Methylbutenol weit unterhalb der Reaktionstemperatur liegt. Die Verwendung eines hochsiedenden Lösungsmittels erniedrigt hingegen die Raum-Zeit-Ausbeute.

In dem Tschechischen Patent 216 '360 (1979) wird beschrieben, daß die Carroll-Reaktion in einem Gemisch aus ungesättigtem Keton und Acetessigsäuremethylester bzw. -ethylester unter Zugabe einer gerade zur Reaktion erforderlichen Menge des ungesättigten Alkohols durchgeführt wird. Dabei wird aus dem Reaktionsgemisch das Kohlendioxid und ein Gemisch aus dem nicht umgesetzten ungesättigten Alkohol und Methanol bzw. Ethanol abdestilliert, das kontinuierlich in einer angekoppelten Destillationskolonne fraktioniert wird. Der α,β-ungesättigte Alkohol, dessen Siedepunkt unter 180°C liegen muß, wird anschließend in die Reaktion zurückgeführt. Es werden bei Reaktionszeiten von 8 Stunden Ausbeuten von ca. 80% erreicht. Diese Vorgehensweise ist nach dem Patent von Vorteil, weil ein Mitreißen der beiden tiefersiedenden Komponenten aus dem Reaktionsgemisch durch das entstehende Kohlendioxid nicht vermieden werden kann.

Die in diesem Patent beschriebene Ankopplung einer Destillationskolonne an das eigentliche Reaktorsystem ist nicht unbedingt erforderlich, da durch richtige Auslegung des Reaktorsystems ein Mitreißen des α,β-ungesättigten Alkohols durch Kohlendioxid vermieden werden kann. Beispielsweise gelingt es in DE 2 928 944, nur Methanol und Kohlendioxid abzutrennen und den α,β-ungesättigten Alkohol in der Reaktionsblase zu halten. Somit resultieren aus der angekoppelten Destillationskolonne in erster Linie zusätzliche Investitions- und Energiekosten. Ein weiterer Nachteil ist die Beschränkung der Siedetemperatur des α,β-ungesättigten Alkohols auf unter 180°C, da die meisten für die Vitamin-E-Synthese relevanten Alkohole oberhalb 200°C sieden.

Im Gegensatz zu den oben genannten Patentschriften wird in der DE 2 928 944 (1979) die Verwendung eines Lösungsmittels beschrieben, dessen Siedepunkt zwischen dem eingesetzten Acetessigsäureester und dem des hieraus abzuspaltenden Alkohols liegt. Dieses Lösungsmittel wird als "Zwischensieder" bezeichnet. Als besonders vorteilhafte Ausführungsform wird die Verwendung von 3-Methyl-1- buten-3-ol als reaktiver Zwischensieder genannt, wobei als zusätzliche erwünschte Nebenreaktion dessen Umsetzung mit dem Acetessigsäurealkylester zu 2-Methyl-2-hepten-6-on als weiterem Wertprodukt stattfindet. Als Vorteile der Verwendung eines solchen Zwischensieders werden erhöhte Produktausbeuten sowie kürzere Reaktionszeiten und damit hohe Raum-Zeit-Ausbeuten genannt. Als Reaktorsystem wird für eine diskontinuierliche Reaktionsführung eine Destillationsblase mit aufgesetzter Fraktionierkolonne und für eine kontinuierliche Reaktionsführung eine beheizte Kesselkaskade vorgeschlagen.

Die Verwendung eines Zwischensieders hat jedoch die folgenden Nachteile. Bei Verwendung eines inerten Zwischensieders verringert sich das für die Edukte zur Verfügung stehende Reaktorvolumen, d.h. die erzielbare Raum-Zeit-Ausbeute verringert sich zwangsläufig. Die Verwendung des reaktiven Zwischensieders 3-Methyl-1-buten-3-ol führt dagegen zu einer Zwangskoppelproduktion von 2-Methyl-2-hepten-6-on, die unerwünscht sein kann. Darüber hinaus ist das Verfahren auf Systeme beschränkt, bei denen der α,β-ungesättigte Alkohol höher siedet als der verwendete Acetessigsäurealkylester.

EP 816 321 beschreibt ein Verfahren zur Herstellung von Hexahydrofarnesylaceton (Phyton) bzw. Isophytol unter anderem über die Stufen Ethinylierung/Partialhydrierung von Ketonen und Carroll-Reaktion. Das dort offenbarte Verfahren weist für die einzelnen Stufen folgende Nachteile auf:
- In der Ethinylierung wird quantitativer Umsatz erzeugt, wodurch nur schlechte Selektivitäten erzielt werden. Die Verweilzeiten sind mit 2-10 h sehr hoch, was geringe Raum-Zeit-Ausbeuten und damit große Reaktoren bedingt. Die Neutralisation wird mit Ammoniumsulfat durchgeführt, was einen zusätzlichen Salzanfall im Abwasser bedeutet.
- Die beschriebene Partialhydrierung weist ebenfalls schlechte Raum-Zeit-Ausbeuten und Selektivitäten auf. Zudem ist sie aufwendig, da mit einem Suspensionskatalysator gearbeitet wird, dessen vollständige Abtrennung vom Produkt nur mit hohem technischen Aufwand realisierbar ist.
- Die Carroll-Reaktion führt in der beschriebenen technischen Ausführung ebenfalls zu für ein technisches Verfahren geringen Selektivitäten und Raum-Zeit-Ausbeuten.

Gegenstand der Erfindung ist ein Verfahren zur Herstellung von höheren, α,β-ungesättigten Alkoholen, das besonders hohe Raum-Zeit-Ausbeuten und Selektivitäten liefert und sich mit geringem technischen Aufwand umsetzen lässt.

Die Aufgabe wurde erfindungsgemäß gelöst durch ein Verfahren zur Herstellung von von höheren, α,β-ungesättigten Alkoholen der allgemeinen Formel Ia bzw. Ib 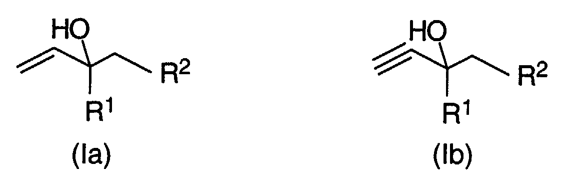 wobei
- R¹: Wasserstoff oder einen C₁-C₄-Alkylrest bedeutet
- R²: die Gruppe der allgemeinen Formel II 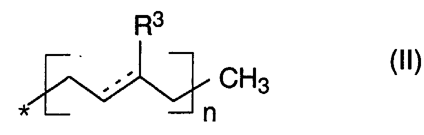 Wasserstoff einen gesättigten oder einen ein- oder mehrfach ungesättigten C₁-C₃₀-Alkyl-, Cycloalkylalkyl- oder Cycloalkylrest bedeuten, die gegebenenfalls mit C₁-C₄-Alkyl substituiert sein können
- R³: Wasserstoff oder einen C₁-C₄-Alkylrest bedeutet,
und die gestrichelte Linie eine zusätzliche Doppelbindung bedeuten kann, durch
- n: die Zahl 0 oder 1 bis 6 bedeutet, durch
a) basekatalysierte Monoethinylierung eines Ketons der allgemeinen Formel R¹-CO-CH₂-R² in flüssigem Ammoniak (NH₃/KOH-Verfahren, bzw. NH₃/MOR-Verfahren), wobei R¹ und R² die oben angegebene Bedeutung besitzen,
b) gegebenenfalls anschließender Hydrierung des Acetylenalkohols der allgemeinen Formel Ib in Gegenwart von Wasserstoff an einem Pd-haltigen Dünnschichtkatalysator und
c) anschließender Reindestillation des Hydrieraustrages, sowie gegebenenfalls
d) Umsetzen des in den Stufen a) bis c) oder a) und c) erzeugten Alkohols der allgemeinen Formel Ia bzw. Ib 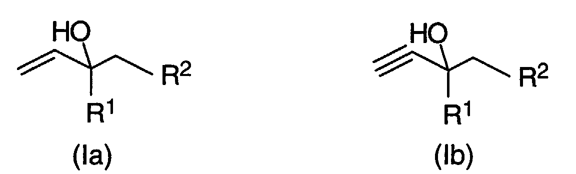 mit Diketen oder Acetessigsäurealkylestern der allgemeinen Formel IV 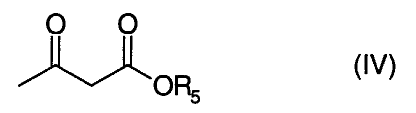 in der R⁵ für C₁-C₄-Alkyl steht,
   in Gegenwart von 0,1 bis 0,5 Mol-% einer organischen Aluminiumverbindung bezogen auf den umzusetzenden Acetessigsäurealkylester zur Herstellung des korrespondierenden um 3 C-Atome verlängerten Methylketons,
e) und Einsatz des gegebenenfalls in Stufe d) gewonnenen Ketons in die Stufen a) bis c),

Der Vorteil des erfindungsgemäßen Verfahrens liegt in der Kombination der Stufen a) mit den Stufen b) und/oder c), sowie gegebenenfalls d) und e). Diese Kombination ist von großem wirtschaftlichen Nutzen, wenn bei den einzelnen Stufen bevorzugt folgende Bedingungen eingehalten werden:

Im Gegensatz zum Stand der Technik wird die Ethinylierung des Ketons, Stufe a), nur bis zu einem Teilumsatz von 50 bis 95 %, vorzugsweise 75 bis 85 % betrieben. Dadurch wird eine besonders hohe Selektivität größer 90 %, vorzugsweise größer 97 % erhalten. Vor allem die Bildung von Diolen, die durch Reaktion von monoethinyliertem Keton mit einem weiteren Mol Keton als selektivitäts- und qualitätsmindernde Nebenreaktion auftritt, findet nur bis zu einem Gehalt von 1 %, vorzugsweise 0 bis 0,3 % statt. Dies ist von besonderer Bedeutung, da die Diole in Stufe b) weitgehend intakt bleiben, bei der anschließenden Destillation nach c) kontinuierlich zum Acetylenalkohol und Keton zurückspalten und den gewonnenen Allylalkohol verunreinigen. Insbesondere bei der Weiterverarbeitung von Isophytol mit Trimethylhydrochinon zu Vitamin E führt Dehydroisophytol zu unerwünschten Nebenprodukten.

Der Vorteil der hohen Selektivität bei Teilumsatz bleibt erhalten, indem die im Reaktionsgemisch enthaltene Ethinylverbindung nach Neutralisation vorzugsweise mit Kohlendioxid in Wasser ohne Aufreinigung nach dem beispielsweise in EP 827 944 beschriebenen Verfahren, Stufe b), zum entsprechenden Allyalkohol umgesetzt wird. Hierbei ist die Selektivität überraschenderweise so hoch, dass das im Gemisch enthaltene Keton trotz einer Konzentration von 5 bis 50 %, vorzugsweise 15 bis 25 % nahezu unverändert und die Bildung der korrespondierenden Alkohole unter 1 %, vorzugsweise 0 bis 0,3 % bleibt. Diese Alkohole stellen Produktverlust dar und mindern den wirtschaftlichen Nutzen, da sie als Ballast die verfügbare Kapazität der Anlage vermindern.

Der Hydrieraustrag, der nach b) erhalten wird und überwiegend aus Allyalkohol und Keton besteht, wird entsprechend c) destillativ getrennt, wobei das in a) nicht umgesetzte Keton abgetrennt und nach a) zurückgeführt wird. Der Nutzen der nach a) und b) erhaltenen hohen Selektivität wird dadurch vergrößert, wenn das Keton in hoher Reinheit erhalten und zurückgeführt wird. Dabei ist zum einen ein geringer Gehalt an korrespondierendem Alkohol essentiell, wie er nach b) erhalten wird. Dieser kann destillativ nur unvollständig und/oder mit sehr hohem technischen Aufwand bei gleichzeitigem Ketonverlust abgetrennt werden, da er zusammen mit dem Keton wieder in die Stufe a) gelangt, sich dort als inerter Ballast aufpegelt und die Kapazität der Anlage vermindert. Dies würde den Vorteil der hohen Selektivitäten aus a) und b) verringern und den wirtschaftlichen Nutzen des Verfahrens vermindern. Das Ausschleusen eines Teiles des Rückführstromes von > 5 % würde wegen des Verlustes an Keton den Vorteil der hohen Selektivitäten der Stufen a) und b) vermindern.

Es ist von großem wirtschaftlichen Nutzen, die destillative Trennung des nach a) und b) erhaltenen Stoffgemisches vorzugsweise in einer Trennwandkolonne z.B. nach EP 0 122 367 bzw. U.S. 2,471,134 vorzunehmen, da so die erforderliche Reinheit von Allylalkohol und Keton mit geringst möglichem technischen Aufwand erreicht werden kann.

Unabhängig von der Destillationsmethode ist es von besonderem Vorteil, den Hydrieraustrag vor der Destillation über eine Kurzwegverdampfung von hochsiedenden Komponenten abzutrennen und nur das von Schwersiedern befreite Destillat in die Seite der Trennkolonne einzutragen. Dies geschieht bei Normaldruck, vorzugsweise bei vermindertem Druck, wobei hier vorteilhaft der niedrigste technisch erreichbare und wirtschaftlich vertretbare Druck angewandt wird. Bei der Kurzwegverdampfung wird am Ort des Verdampfungsvorganges der niedrigst mögliche Druck mit minimalem Druckverlust erzielt, da zwischen Vakuumaggregat und Verdampferfläche keine Druckverlust verursachenden Einbauten benötigt werden und hinreichend große Leitungsquerschnitte verwendet werden können. Ein verminderter Druck von 0,1 bis 20 mbar, vorzugsweise 1 bis 2 mbar, ist problemlos zu erreichen. Ein besonders vorteilhafter Aufbau ist die gleichzeitige Nutzung dieses Kurzwegverdampfers für die Schwersiederabtrennung aus dem Destillationssumpf der Trennkolonne, indem ein Teilstrom des Destillationssumpfes dem frischen Zulauf zum Kurzwegverdampfer zugeführt wird und gemeinsam von den Schwersiedern befreit wird. Der besondere Vorteil besteht darin, dass die Destillationskolonne nur mit unter den Destillationsbedingungen verdampfbaren Komponenten belastet wird und die Siedetemperatur im Sumpf minimal gehalten werden kann. Dies ist von besonderem wirtschaftlichen Nutzen, da insbesondere bei hochsiedenden Gemischen die thermische Belastung minimiert werden kann und damit auch die Verluste durch thermische Zersetzung bzw. Kondensation zu schwersiedenden Bestandteilen. Dies gilt insbesondere für den Austrag der Hydrierung nach b), wenn 2-Nerolidol, Dihydronerolidol oder Tetrahydronerolidol in Gegenwart der in a) eingesetzten Ketone destilliert wird, ebenso für Isophytol in Gegenwart des nach a) eingesetzten Hexahydrofarnesylacetons.

Die anschließende Kombination mit der Stufe (d) und die Verwendung des Austrags aus der Stufe (d) als Einsatzstoff für die Stufe (e) ist nach dem erfindungsgemäßen Verfahren besonders vorteilhaft, da das Keton aus der Stufe d) aufgrund des dort offenbarten Verfahrens einen besonders geringen Gehalt an dem durch Meerwein-Ponndorf-Verley-Reduktion entstehenden Alkohols enthält. Die vorteilhaften Auswirkungen dieses niedrigen Alkohol-gehaltes wurden bereits oben bei der Kombination der Stufen a), b) und c) dargelegt. Dieser niedrige Gehalt an Alkohol macht zudem den sequentiellen Aufbau längerer, um jeweils eine Pentan-2-on-5-yliden-Gruppe erweiterter Allylakohole nach d) und e) wirtschaftlich besonders interessant, da wegen der geringen Nebenproduktrate innerhalb dieser Sequenz jeder um C₅, C₁₀, C₁₅ etc. gewünschte Alkohol in einer guten Ausbeute erhalten wird.

Das Verfahren kann auch so durchgeführt werden, daß ein um eine Pentan-2-on-5-yliden-Gruppe erweiterter Alkohol der allgemeinen Formel Ia bzw. Ib in Abfolge der Schritte d), und e) erhalten wird.

Ein weiterer Gegenstand der Erfindung ist ein Verfahren zur Herstellung eines Alkohols der allgemeinen Formel Ia, das dadurch gekennzeichnet ist, daß nur die Stufen a) bis c) durchlaufen werden.

In einer weiteren erfindungsgemäßen Verfahrensvariante wird der in Stufe a) gewonnene Acetylenalkohol der allgemeinen Formel Ib unter Umgehung der Stufe b) direkt der Reindestillation gemäß Stufe (c) unterworfen. Im Anschluß daran kann eine weitere Umsetzung nach den Stufen d) und e) erfolgen.

Bevorzugte erfindungsgemäße Verfahrensvariante ist jedoch die Herstellung eines Allylalkohols der allgemeinen Formel I durch die Umsetzung der Stufen a) bis e), wobei die Sequenz der Stufen a) bis e) auch mehrfach durchlaufen werden kann.

Vorzugsweise bedeutet bei dem erfindungsgemäßen Verfahren R² eine Gruppe der allgemeinen Formel II

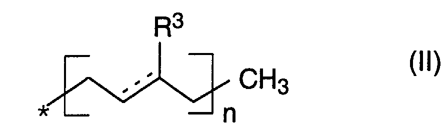

Unter einem C₁-C₄-Alkylrest versteht man einen Methyl-, Ethyl-, Propyl-, i-Propyl-, Butyl- oder einen t-Butylrest.

Unter einem Cycloalkylrest versteht man einen 3- bis 7-gliedrigen Ring, der ein- oder mehrfach ungesättigt sein kann, beispielsweise einen Cyclopropyl-, Cyclobutyl-, Cyclopentyl-, Cyclohexyloder einen Cycloheptenylrest.

Unter einem Cycloalkylalkylrest versteht man einen über einen C₁-C₃₀-Alkyrest gebundenen Cycloalkylrest.

Das Verfahren kann sowohl kontinuierlich als auch im Batch-Verfahren durchgeführt werden. Bevorzugt ist jedoch die kontinuierliche Fahrweise.

Unter den höheren, α,β-ungesättigten Alkoholen der allgemeinen Formel Ia versteht man Alkylalkohole, unter den Alkoholen der Formel Ib Propargylalkohole, vorzugsweise werden mit dem erfindungsgemäßen Verfahren Alkylalkohole der allgemeinen Formel Ia hergestellt.

Bevorzugt sollten die als Zwischenprodukte für die Herstellung von dem essentiellen Vitamin-E-Vorprodukt Isophytol, die für Riech- und Aromastoffe begehrten Allylalkohole wie 3,7,11-Trimethyl-1,6,10-dodekatrien-3-ol (2-NER), 3,7,11-Trimethyl-1,6-dodekadien-3-ol (HNER), 3,7,11-Trimethyldodec-1-en-3-ol (THNER), 3,7,11,15-Tetramethyl-1-hexadecen-3-ol (IP), 3,7-Dimethylocta-1,6-dien-3-ol (2-LIN), 3,7-Dimethyloct-1-en-3-ol (HLIN), 3-Methyl-1-(2,6,6-trimethyl-1-cyclohexen-1-yl)-penta-1,4- dien-3-ol, 3-Methyl-3-hydroxybuten (MBE) bzw. die Acetylenalkohole 3,7,11-Trimethyldodec-1-yn-3-ol (TMD) 3,7,11,15-Tetramethyl-hexadec-1-yn-3-ol (DIP), 3,7,11-Trimethyl-6,10-dodekadien-1-yn-3-ol (2-DHNER), 3,7,11-Trimethyl-6-dodecen-1-yn-3-ol (DHNER), 3,7-Dimethylocta-6-en-1-yn-3-ol (2-DHL), 3,7-Dimethyloct-1-yn-3-ol (HDHL), 3-Methyl-1-(2,6,6-trimethyl-1-cyclohexen-1-yl)-penta-4-en-1-yn-3-ol, 3-Methyl-3-hydroxybutin (MBI), besonders bevorzugt die Alkohole 3,7,11-Trimethyldodec-1-yn-3-ol (TMD) 3,7,11,15-Tetramethyl-hexadec-1-yn-3-ol (DIP), 3,7,11-Trimethyldodec-1-en-3-ol (THNER), 3,7,11-Trimethyl-1,6,10-dodekatrien-3-ol (2-NER), 3,7,11-Trimethyl-6,10-dodekadien-1-yn-3-ol (2-DHNER) und 3,7,11,15-Tetramethyl-1-hexadecen-3-ol (IP) mit höherer Selektivität, höheren Raum-Zeit-Ausbeuten und auf technisch einfache Weise hergestellt werden.

Des weiteren können auch Ketone in die Ethinylierung nach (a) eingesetzt werden, die durch partielle oder vollständige selektive C=C-Hydrierung von 6,10-Dimethylundeca-3,5,9-trien-2-on (Pseudojonon, PSJ) erhalten oder 6,10,14-Trimethylpenta-5,9,13- trien-2-on (2-Farnesylaceton) erhalten werden.

Weitere Beispiele für die Ketone der allgemeinen Formel IV, außer den für die Herstellung der oben genannten Alkohole entsprechenden Ketonen sind Aceton, Diethylketon, Methylethylketon, Cyclohexanon, Methylisobutylketon, Methylvinylketon.

Prinzipiell können alle durch Ethinylierung in der Stufe a) hergestellten Acetylenalkohole selektiv zu den entsprechenden Allylalkoholen hydriert werden.

Die Acetylenalkohole können als Reinsubstanzen oder gemischt miteinander in der Hydrierung umgesetzt werden. Des weiteren stört es die Hydrierung nicht, wenn in Stufe a) nicht umgesetztes Keton im Gemisch mit dem entsprechenden Acetylenalkohol vorliegt, da unter den erfindungsgemäßen Verfahrensbedingungen das Keton auch in hoher Konzentration in der Hydrierung nicht angegriffen wird.

Trennwandkolonnen und thermisch gekoppelte Kolonnen eignen sich nach dem erfindungsgemäßen Verfahren insbesondere zur destillativen Auftrennung des Reaktionsaustrags aus Stufe a) und b) und Stufe d). Bei der Aufarbeitung des Reaktionsaustrags aus Stufe a) und b) wird das Wertprodukt, der Allylalkohol, über den Seitenabzug der Kolonne in hoher Reinheit entnommen.
Das nicht umgesetzte Keton fällt am Kopf oder an einem im oberen Drittel der Kolonne liegenden Seitenabzug an und wird, gegebenenfalls nach Ausschleusung eines Teilstroms, in Stufe a) zurückgeführt. Hochsiedende Nebenprodukte werden über den Sumpf der Kolonne ausgeschleust.

Alternativ kann die Trennwandkolonne mit 2 Seitenabzügen versehen werden. Auf diese Weise können Nebenprodukte, die einen Siedepunkt zwischen Wertprodukt und Leichtsiedern oder zwischen Wertprodukt und Hochsiedern haben, in hoher Konzentration ausgeschleust werden.

So zum Beispiel kann bei der Herstellung von H-Nerolidol das Wertprodukt über den Seitenabzug in einer Reinheit von über 98 % entnommen werden. Das nicht umgesetzte H-Geranylaceton fällt am Kopf der Kolonne an und kann in Stufe a) zurückgeführt werden. Über den Sumpf der Kolonne werden die Hochsieder ausgeschleust. Es ist besonders vorteilhaft, die Schwersieder aus dem Sumpf der Destillationskolonne kontinuierlich an einem außenliegenden Kurzwegverdampfer abzutrennen, der bei einem im Vergleich zur Destillationskolonne niedrigerem Druck betrieben wird. Das Destillat des Kurzwegverdampfers wird wieder in die Trennwandkolonne zurückgeführt. Dadurch kann eine minimale Sumpftemperatur erreicht werden, was die Zersetzung von Wertprodukt minimiert und die Destillationsausbeute erhöht.

Die Destillation nach c) kann alternativ zu Trennwandkolonnen auch in jeweils zwei thermisch gekoppelten Kolonnen durchgeführt werden. werden. Bei thermisch gekoppelten Kolonnen kann es von Vorteil sein, den Sumpfstrom der ersten Kolonne in einem zusätzlichen Verdampfer teilweise oder vollständig zu verdampfen und danach der zweiten Kolonne zuzuführen. Diese Vorverdampfung bietet sich insbesondere bei dem vorliegenden Fall an, weil der Sumpfstrom der ersten Kolonne größere Mengen an Mittelsieder enthält. In diesem Fall kann die Vorverdampfung auf einem niedrigeren Temperaturniveau erfolgen und der Verdampfer der zweiten Kolonne entlastet werden. Weiterhin wird durch diese Maßnahme der Abtriebsteil der zweiten Kolonne wesentlich entlastet. Der vorverdampfte Strom kann dabei der zweiten Kolonne zweiphasig oder in Form von zwei separaten Strömen zugeführt werden.

Darüber hinaus kann es sowohl bei Trennwandkolonnen als auch bei thermisch gekoppelten Kolonnen von Vorteil sein, den Zulaufstrom einer Vorverdampfung zu unterziehen und anschließend zweiphasig oder in Form von zwei Strömen der Kolonne zuzuführen. Diese Vorverdampfung bietet sich besonders dann an, wenn der Zulaufstrom größere Mengen an Leichtsiedern enthält. Im vorliegenden Fall ist dies bei geringen Umsätzen der Fall. Durch die Vorverdampfung kann der Abtriebsteil der Kolonne wesentlich entlastet werden.

Trennwandkolonnen und thermisch gekoppelte Kolonnen können sowohl als Packungskolonnen mit Füllkörpern oder geordneten Packungen oder als Bodenkolonnen ausgeführt werden. Bei den Packungskolonnen sind geordnete Blechpackungen mit einer spezifischen Oberfläche von 100 bis 500 m²/m³, bevorzugt etwa 250 bis 300 m²/m³ besonders geeignet.

Die Herstellung von beispielsweise Dehydro-Dihydronerolidol (DHNER) (Stufe a)) und den anderen Acetylenalkoholen erfolgt vorzugsweise kontinuierlich (20 bar, 20 bis 50°C; NH₃ (1) als Lösungsmittel). Besonders vorteilhaft ist es, die Ethinylierung in einem nicht rückvermischten Rohrreaktor mit Plug-Flow-Charakteristik durchzuführen. Diese Charakteristik ist gegeben, wenn die Kesselzahl (Definition s. Baerns, Hofmann, Renken, Chemische Reaktionstechnik, Lehrbuch der Technischen Chemie, Band 1, 2. Auflage, Thieme Verlag Stuttgart 1992, S. 328-330) größer als 50 ist. Der Reaktionsaustrag wird in einem Mischorgan wie einem statischen Mischer oder einer Reaktionsmischpumpe, wie in DE 4220239 beschrieben, hydrolysiert und neutralisiert. Die Phasentrennung erfolgt mit Hilfe eines Koaleszensfilters. Die Abtrennung des Restwassers aus der organische Roh-DHNER-Lösung (Abreicherung von 1,5 Gew.-% auf > 0,5 Gew.-%) erfolgt abschließend in einem unter Vakuum betriebenen Dünnschichtverdampfer.

DHNER wird in einer einstufigen Reaktion aus HGAC und Acetylen mit Hilfe katalytischer Mengen Base gebildet. Die Base, allgemein als MOR bezeichnet, kann bestehen aus Alkalihydroxiden, gelöst in einem aliphatischen C₁-C₈-Alkohol, wie Methanol, Ethanol, Propanol, i-Propanol, Butanol, bevorzugt KOH in Methanol, Alkalialkoholaten in dem entsprechenden Alkohol, bevorzugt Kaliummethylat in Methanol oder einem beliebigen primären, sekundären oder tertiären Amin, besonders bevorzugt aber besteht die Base aus Kaliummethylat in Methanol.

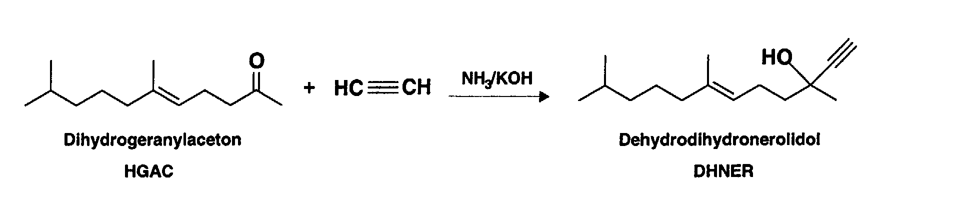

Der Ethinylierungsteil besteht aus einem Rohrreaktor mit drei flüssigen und einem gasförmigen Zulauf, die in einem Mischer innig vermischt werden. Ammoniak, das Keton und die basische Katalysator-Lösung werden kontinuierlich in den Reaktor gefördert. Acetylen wird geregelt in den Reaktor dosiert. Besonders vorteilhaft ist es, wenn ohne gasförmigen Zulauf gearbeitet wird, indem das benötigte Acetylen in einem vorgeschalteten Sättiger in Ammoniak eingelöst und als ammoniakalische Lösung dem Mischer zugeführt wird. Eine weitere bevorzugte Ausführungsform besteht darin, den durch die Reaktion verbrauchten Acetylenanteil nach einer Teilstrecke des Reaktors durch Nacheinspeisung von Acetylen bzw. von Acetylen gelöst in Ammoniak zu ersetzen. Die Reaktion findet bei 20 bar Druck und Temperaturen von ca. 20 - 50°C statt.

Das molare Verhältnis der Reaktanden wird bevorzugt wie folgt eingestellt:

| **Keton** | **Acetylen** | **Base** | **NH3** |
|---|---|---|---|
| Mol:mol | mol:mol | mol:mol | mol:mol |
| 1 | 2.5-3.5 | 0.01-0.03 | 12-25 |

Der Umsatz an Keton in der Ethinylierung kann vollständig sein, die Ethinylierung gelingt aber mit besonderem Vorteil, wenn nur ein Teilumsatz zugelassen wird.

Das in der Reaktion eingesetzte Ammoniak und das nicht in der Reaktion verbrauchte Acetylen werden zurückgeführt. Die Abtrennung des Ammoniaks und des Acetylens erfolgt gemeinsam aus der Reaktionsmischung durch Flashverdampfung.

Die Abgasströme werden gesammelt und über eine Absorptions- und Desorptionskolonne geleitet. Über diese Kolonnen wird Ammoniak aus dem Abgasstrom mit Wasser oder niedrigen Glykolen wie z.B. Ethylenglykol vom Acetylen abgetrennt und destillativ in reiner Form wiedergewonnen, während das nicht verbrauchte Acetylen erneut verdichtet und in die Reaktion zurückgeführt wird.

Die entgaste organische Rohlösung wird zur Neutralisation in ein Mischaggregat wie einen statischen Mischer oder eine Reaktionsmischpumpe dosiert. Darin wird der Rohaustrag mit Wasser und CO₂ intensiv vermischt und neutralisiert, wobei hier unter Normaldruck als auch unter erhöhtem Druck gearbeitet werden kann. Im ersten Fall liegt während des Neutralisationsprozesses ein 3-phasiges Gemisch vor: organische Phase, wässrige Phase und Gasphase, während unter Druck die Neutralisation in einem 2-phasigen Gemisch - organische Phase (flüssig) und wässrige Phase (flüssig) vorliegt. Vorzugsweise wir die 2-phasige Verfahrensvariante unter Druck angewendet, da dadurch der Prozess besser steuerbar und kein nennenswerter Überschuss an Gasphase benötigt wird. Der Austrag wird in wässrige und organische Phase getrennt. Die organische Phase wird zur Entwässerung über einen Dünnschichtverdampfer geleitet und das restliche Wasser über Kopf abgezogen. Der entwässerte Austrag, der beispielsweise neben DHNER auch das nicht umgesetzte Keton HGAC enthalten kann, wird direkt in die Hydrierung weitergeleitet.

HNER wird in einer einstufigen Reaktion aus durch Umsetzung des DHNER mit Wasserstoff an einem Pd-haltigen Dünnschichtkatalysator gewonnen (Stufe b)). Das im Feed enthaltene Keton wird dabei nicht angegriffen. Der Dünnschichtkatalysator kann durch Bedampfen, Besputtern (EP564830, EP 412415) oder vorzugsweise durch Tränkung (EP 827 944) hergestellt werden. Als Aktivkomponenten sind die in EP 827 944 genannten in den dort angegebenen Konzentrationen sinnvoll. Die äußere Form der Katalysatoren ist ebenfalls in EP 827 944 beschrieben. Zur Selektivitätssteigerung wird dem Wasserstoff CO zugemischt.

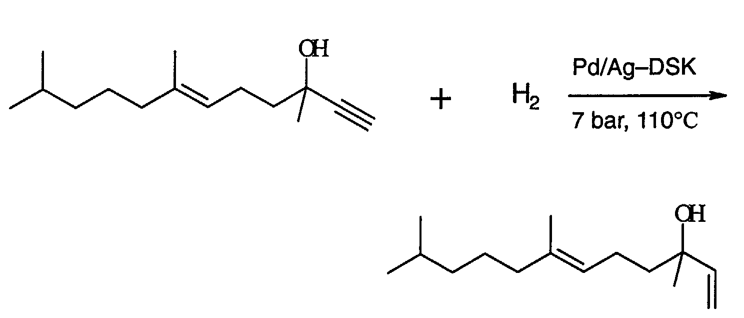

Nach der Reduktion des Katalysators mit Wasserstoff bei Temperaturen von 20 bis 250°C, vorzugsweise 70 bis 200°C, die man vorteilhaft im Reaktor durchführt, ist der Katalysator für die erfindungsgemäße Partialhydrierung einsatzbereit.

Die verwendeten Katalysatoren besitzen neben guter Selektivität und Aktivität auch eine hohe Standzeit. Insbesondere für kontinuierlich betriebene Einstranganlagen ist dieses von großem Vorteil, da dadurch Abstellzeiten für häufigen Katalysatorwechsel minimiert werden.

Die Umsetzung des DHNER zum HNER findet in mindestens zwei hintereinander geschalteten Reaktoren statt. Im ersten und gegebenenfalls in einem weiteren mit Flüssigkreislauf und Gaskreislauf ausgestatteten Reaktor werden 90 bis 95 % des Umsatzes und im zweiten und gegebenenfalls in einem weiteren Reaktor mit plug-flow-Charakteristik der Restumsatz erzielt. Der Zulauf zum zweiten Reaktor erfolgt standgeregelt aus dem Gas-Flüssigabscheider im Kreislauf des ersten Reaktors. Mit Vorteil gelingt die Hydrierung, wenn man die Querschnittsbelastungen des Gases und der Flüssigkeit im ersten Reaktor im Bereich von 20 bis 500 m³/m² h, vorzugsweise 100 bis 300 m³/m²*h einstellt. Jeder der beiden Reaktoren kann auch doppelt ausgeführt werden, wobei diese sowohl seriell als auch parallel betrieben werden können.

Mit besonderem Vorteil gelingt die Partialhydrierung in technischem Maßstab, wenn das Kreisgas mittels des Flüssigkeitsstromes und einer geeigneten Vorrichtung, wie einem Flüssigkeits-Gas-Verdichter in feinster Verteilung in den Reaktor eindüst. Zusammen mit der Formgebung der Katalysatormonolithe und der beschriebenen Begasung des Reaktors erzielt man hohe Raum-Zeit-Ausbeuten durch optimale Quervermischung und gute Hydrodynamik an der Katalysatorgrenzfläche. Die Partialhydrierungen werden, je nach Substanz, bei Temperaturen von 20 bis 250°C, vorzugsweise 60 bis 200°C durchgeführt. Die Hydrierung wird im Bereich von 0,3 bis 200 bar, vorzugsweise 0,5 bis 20 bar betrieben.

Die Gasversorgung der beiden Reaktoren mit Wasserstoff und Oxogas (Gemisch aus Wasserstoff und CO 1:1) erfolgt mengengeregelt. Über eine kontinuierliche IR-Messung läßt sich der CO-Gehalt im Abgasstrom bestimmen. Dieser Wert kann zur Regelung des Zuflusses an Oxogas verwendet werden. Besonders vorteilhaft kann man die Hydrierung betreiben, wenn man über eine Umsatzmessung den Zufluss an CO regelt. Die Gehalte an CO im Wasserstoff sollten im Bereich von 10 bis 2000 ppm, vorzugsweise 300 bis 1800 ppm CO liegen. Man kann das CO in der flüssigen Phase auch durch geringfügige Zersetzung einer dem Acetylenalkohol zugemischten Verbindung, die CO abspaltet, entstehen lassen, mit der Maßgabe, daß die oben angegebenen Bereiche der CO-Konzentration in der Gasphase nicht überschritten werden.

Der Austrag aus der Hydrierung (b) wird dann direkt in die destillative Aufarbeitung hineingefahren (Stufe (c)).

Die Reindestillationskolonne ist eine Packungskolonne mit Trennwand und wird bei einem Kopfdruck von 20 bis 300 mbar, vorzugsweise 150 mbar betrieben.

Das hydrierte Gemisch wird in den seitlichen Zulauf der Kolonne gegeben. Über Kopf gehen die Leichtsieder - hauptsächlich unumgesetztes Keton ex Stufe 2 (hier HGAC) - und werden im Kopfkondensator oder über den obenliegenden Seitenabzug auskondensiert. Die auskondensierten Brüden werden aufgefangen und in die Ketonvorlage der Stufe (a) rückgeführt. Die Möglichkeit zur Ausschleusung ist vorgesehen. Vorteilhafter kann das Keton in höherer Reinheit aus einem zweiten Seitenabzug im oberen Bereich der Trennwandkolonne abgezogen werden, wobei über Kopf ein geringerer Strom von Leichtsiedern erhalten wird.

Das gereinigte HNER wird im Bereich der Trennwand im Seitenabzug flüssig gewonnen.

Im Sumpf der Kolonne werden Temperaturen von maximal 200°C, vorzugsweise 180°C bis 190°C erreicht. Der Sumpfwärmetauscher wird mit 16 bar Dampf betrieben. Bei diesen Temperaturen sind nur noch geringe Mengen HNER im Sumpf enthalten. Der Sumpf wird zur Abtrennung dieses HNER-Gehaltes in einen Dünnschichtverdampfer gegeben. Dessen Kopfabzugsprodukt wird in die Kolonne zurückgefahren. Der Sumpf des Dünnschichtverdampfers wird aus dem Verfahren ausgeschleust.

Setzt man anschließend das nach den Stufen a)-c) gewonnene H-NER gegebenenfalls nach Stufe d) weiter zum H-Farnesylaceton (6,10,14 Trimethyl-5,9-pentadecadien-2-on) um, erfolgt die Herstellung vorteilhaft kontinuierlich in einer Reaktionskolonne bei 500 mbar. Über Kopf der Reaktionskolonne werden das entstehende Methanol und Kohlendioxid abgetrennt. Der Sumpfstrom der Reaktionskolonne, der das Wertprodukt zusammen mit Hochsiedern enthält, wird in einer bei 100 mbar betriebenen Trennwandkolonne aufgearbeitet. Der Sumpf der Trennwandkolonne wird in einem nachgeschalteten Dünnschichtverdampfer von restlichem Wertprodukt befreit, das zur Erhöhung der Ausbeute in die Kolonne zurückgeführt wird. Nicht umgesetzte Reaktanden werden in die Reaktionskolonne zurückgeführt.

Das H-Farnesylaceton wird durch Umsetzung von H-NER mit Acetessigsäuremethylester (AME) nach einer sogenannten Carroll-Reaktion hergestellt, bei der Methanol und Kohlendioxid als Nebenprodukte entstehen. Die Reaktion kann zwar thermisch durchgeführt werden, jedoch werden in Gegenwart von Aluminiumalkoholaten bzw. Aluminiumtriacetoacetaten wesentlich höhere Ausbeuten erzielt.

Als Nebenreaktion der H-Farnesylaceton-Synthese läuft eine Meerwein-Ponndorf-Verley-Reduktion ab, bei der die H-Farnesylaceton-Isomere zu den entsprechenden Alkoholen reduziert werden.

Die sogenannten H-Farnesylaceton-ole sind unerwünschte Nebenkomponenten. Die Meerwein-Ponndorf-Verley-Reduktion wird durch Aluminiumalkoholate katalysiert. Aus diesem Grund ist bei der Carroll-Reaktion der Einsatz von Aluminiumtriacetoacetaten von Vorteil.

Als weitere Nebenreaktion ist die thermische Dehydratisierung von H-NER zu nennen, bei der verschiedene Dimethyldodecadiene entstehen.

Neben oben genannten Nebenprodukten werden bei der Carroll-Reaktion Zersetzungs- und Folgeprodukte des Acetessigsäuremethylesters beobachtet. So zum Beispiel findet man Aceton, Methylacetat und Dehydrazetsäure.

Das erfindungsgemäße Verfahren wird zweckmäßigerweise so durchgeführt, daß man die Reaktanden zusammen mit dem Katalysator in eine Fraktionierkolonne dosiert. Dabei ist es von Vorteil, wenn man den höher siedenden Reaktanden (H-NER) getrennt oder zusammen mit dem flüssigen Katalysator oberhalb des tiefer siedenden Reaktanden (AME) kontinuierlich in die Fraktionierkolonne einspeist. In der Fraktionierkolonne findet nun die Reaktion mit einer überlagerten Destillation statt. Dadurch werden das während der Reaktion freiwerdende Kohlendioxid und der aus dem Acetessigsäurealkylester stammende Alkohol (Methanol) ständig aus dem Reaktionsgemisch entfernt.

Das Kohlendioxid und das Methanol verlassen die Kolonne zusammen mit den Leichtsiedern über den Kopfstrom und gelangen in den Kondensator, wo die kondensierbaren Bestandteile des Brüdenstroms auskondensiert werden. Ein Teil des Kondensats wird als Rücklauf auf die Kolonne gegeben und der andere Teil abgezogen. Es sollte ein Rücklaufverhältnis zwischen 1 und 10, vorzugsweise zwischen 2 und 4 eingestellt werden. Es ist aber auch möglich, das Kondensat vollständig abzuziehen, wenn der höhere siedende Reaktand auf eine der obersten Stufen aufgegeben wird. Der Druck am Kolonnenkopf wird so eingestellt, daß die Temperatur im Sumpf zwischen 100 und 300°C, bevorzugt zwischen 180 und 220°C liegt. Je nach Stoffsystem und gewünschter Sumpftemperatur kann dies mit einer Vakuumpumpe und/oder einem Regelventil geschehen. Das Reaktionsprodukt sammelt sich im Sumpf der Kolonne und wird mittels einer Pumpe zusammen mit den nicht umgesetzten Reaktanden (H-NER, AME) über den Sumpfstrom abgezogen. Das Rohprodukt (H-Farnesylaceton) wird mit Hilfe eines Regelventils über die Produktleitung ausgeschleust und der weiteren Aufarbeitung zugeführt.

Die Dosiermengen werden so gewählt, daß das stöchiometrische Verhältnis der Reaktanden zwischen 0,8 und 1,2, vorzugsweise zwischen 0,95 und 1,05 liegt und sich ein Katalysatorgehalt von 0,1 bis 5 mol-%, vorzugsweise von 1 bis 3 mol-% bezogen auf den umzusetzenden Acetessigsäurealkylester einstellt. Die Verweilzeit des Reaktionsgemisches im Reaktorsystem bestehend aus Verdampfer und Fraktionierkolonne sollte 15 Minuten bis 6 Stunden, vorzugsweise 0,5 bis 2 Stunden betragen. Zur Durchführung der Umsetzung sind 10 bis 100 theoretische Stufen, vorzugsweise zwischen 20 und 40 Stufen erforderlich. Im oberen Teil der Kolonne sollten 0 bis 5 Stufen über dem Zulaufstrom und im unteren Teil der Kolonne 0 bis 5 Stufen unterhalb des Zulaufstroms vorgesehen werden.

Bei der Aufarbeitung des Rohproduktstroms ist es von Vorteil, zunächst den Katalysator mit Hilfe eines Dünnschichtverdampfers abzutrennen, wobei vorzugsweise auch ein Teilstrom des Sumpfes der nachfolgenden Destillations- oder Trennwandkolonnen zur Abtrennung der dort entstandenen Schwersiedern dem Zustrom beigemischt wird, und anschließend das Wertprodukt in nachfolgenden Destillationskolonnen zu isolieren. Nicht umgesetzte Reaktanden (H-NER, AME), abgetrennt in den nachfolgenden Destillations- oder Trennwandkolonnen, können in die Reaktionskolonne zurückgeführt werden. Bevorzugt ist der Einsatz einer Trennwandkolonne, bei der über Kopf H-NER und AME, über einen Seitenabzug das H-Farnesylaceton und über Sumpf die Hochsieder abgetrennt werden. Es ist ebenfalls möglich, eine Trennwandkolonne mit 2 Seitenabzügen zu verwenden. In diesem Fall wird das Wertprodukt über den unteren Seitenabzug und der nicht umgesetzte Allylalkohol H-NER über den oberen Seitenabzug entnommen.

Alternativ ist es möglich, den Sumpfstrom der Reaktionskolonne direkt in der Trennwandkolonne aufzuarbeiten. In diesem Fall sollte der Sumpfstrom in einen nachgeschalteten Dünnschichtverdampfer geleitet werden, in welchem die Abtrennung der Hochsieder erfolgt. Der Brüdenstrom des Dünnschichtverdampfers wird in die Trennwandkolonne zurückgeführt. Das so gewonnene H-Farnesylaceton kann dann wieder in die Stufe a) zur weiteren Umsetzung zum Isophytol eingesetzt werden (Stufe e)).

Die folgenden Beispiele sollen die Erfindung näher erläutern, ohne sie jedoch darauf zu beschränken:
Herstellung von H-Nerolidol

### Stufe (a): Ethinylierung (kontinuierlich betriebene Anlage)

Synthese von Dehydro-Dihydro-Nerolidol (DHNER) durch kontinuierliche Ethinylierung von Hexageranylaceton (HGAC) nach dem NH₃/KOH - Verfahren.

Als Reaktor dient ein 1,8 1 Edelstahlreaktor mit 'Plug-Flow-Charakteristik' (Reaktionsrohr mit 6 mm Innendurchmesser).

In den Reaktor werden nach einer Anfahrphase kontinuierlich 870 g/h HGAC, 285 nl/h Acetylen, 750 g/h NH₃ und 58 g/h Kaliumhydroxid-Lösung in Methanol (8,5 mol-%) gepumpt. Die Dosierung aller drei Ströme erfolgt mengengeregelt in den Reaktor. Die Reaktionstemperatur wird auf 25 bis 35°C temperiert.

Von dem eindosierten HGAC stammen ca. 190 g/h von der Rückführung aus der Destillation nach der Hydrierung.

Die Verweilzeit im Reaktor beträgt 42 min.

Der Reaktionsaustrag erfolgt druckgeregelt (20 bar +/-0,05 bar). Die Entgasung erfolgt in drei Stufen:
1. Flash-Topf bei 90°C; 1013 mbar
2. Dünnschichtverdampfer bei 50°C; 1013 mbar
3. Entgaser bei 40°C; 150 mbar

Die Neutralisation und Hydrolyse erfolgt mit 500 g/h Wasser und 10 nl/h CO₂-Gas in einer Reaktionsmischpumpe bei 70°C. Nach Phasentrennung in einem Koaleszenzfilter (50 µm) bei 70°C erfolgt die Trocknung der organischen Phase in einem weiteren Dünnschichtverdampfer, der bei 90°C und 150 mbar betrieben wird. Es werden kontinuierlich 950 +/-30 g/h organischer Rohaustrag in die Stufe 3 (Hydrierung) weitergeleitet (~ 15 Gew.% unumgesetztes HGAC und ~82 Gew.-% DHNER). Die wäßrige Phase enthält neben Kaliumhydrogencarbonat noch Spuren an Ammoniumhydrogencarbonat (> 0,5 g/100 g).

### Stufe (b) Hydrierung

### Katalysatorherstellung

Ein glattes Kanthalgewebe (Werkstoffnummer 1.4767) mit einer Maschenweite von 180 µm und einem Drahtdurchmesser von 112 µm wird 5 Stunden (h) bei 950°C an der Luft getempert. Ein 20 cm breiter Gewebestreifen wurde auf eine Wickelvorrichtung aufgespannt und anschließend kontinuierlich durch eine Tränkbad transportiert, das eine wässrige Metallsalzlösung aus Palladiumnitrat und Silbernitrat enthielt. Das daraufhin getrocknete Gewebeband hatte eine Beschichtung von 280 mg Pd/m² und 70 mg Ag/m². Das Katalysatorvorprodukt wurde 3 Stunden (h) bei 300°C in einer Elektromuffel formiert. Anschließend wurde das Gewebe gewellt, aufgewickelt und somit zu Monolithen verformt.

Kontinuierliche selektive Hydrierung von Dehydrodihydronerolidol (3,7,11-Trimethyl-6-dodecen-1 in-3-ol) zu Dihydronerolidol (3,7,11-Trimethyl-1,6-dodekadien-3-ol)

Es wurden 4 Metallmonolithen mit 35 mm Durchmesser und 200 mm Höhe und ein Monolith mit 35 mm Durchmesser und 100 mm Höhe in den ersten Rohrreaktor der Anlage eingefüllt. Ein zweiter Rohrreaktor wurde mit 4 Monolithen vom Durchmesser 27 mm und 200 mm Höhe befüllt. Der erste Reaktor wurde in Sumpffahrweise unter Rückführung mit einer Flüssigkeitsquerschnittsbelastung von 200 m³/m² *h und einer Wasserstoffquerschnittsbelastung von 200 m³/m² *h bei einem Gesamtdruck von 7 bar betrieben. Die Eindüsung des Kreisgases in den Reaktor wurde über eine Treibstrahldüse vorgenommen. Dem Wasserstoff wurde soviel CO zudosiert, daß das Abgas, welches in der Zusammensetzung dem Kreisgas entspricht, eine CO-Konzentration von 1200 bis 1500 ppm enthielt. Die Temperatur im ersten Reaktor betrug 115°C. Die Zufuhrmenge an organischem Rohaustrag waren 950 +/-30 g/h. Der zweite Reaktor wird in Sumpffahrweise im geraden Durchgang bei einem Druck von 4 bar, einer Temperatur von 91°C und einer CO-Konzentration im Wasserstoff von 225 ppm betrieben. Die Zufuhrmenge zum zweiten Reaktor wird über den Stand des Gas-Flüssigabscheiders im Kreislauf des ersten Reaktors geregelt. Dessen Austrag wird kontinuierlich in die destillative Aufarbeitung weitergeleitet. Der Austrag enthält ca. 15 Gew.-% unumgesetztes HGAC aus Stufe 2, ca. 78,5 Gew.-% HNER, 2 % unbekannte hochsiedene Komponenten und 1 % Tetrahydronerolidol (überhydrierter Alkohol).

Auch nach 4800 h Betriebszeit verändert der Katalysator seine Performance nicht.

### Stufe (c) Reindestillation

Die Reindestillation erfolgte in einer Trennwandkolonne mit 30 theoretischen Stufen und einem Durchmesser von 80 mm. Als Kolonneneinbauten wurden Packungen des Typs Montz A3-1000 verwendet. Die Kolonne wurde bei einem Kopfdruck von 150 mbar betrieben.

Das hydrierte Gemisch aus Stufe (b) wurde kontinuierlich in den seitlichen Zulauf der Kolonne auf Stufe 14 gegeben. Über Kopf wurden die Leichtsieder, hauptsächlich HGAC, abgetrennt und im Kopfkondensator auskondensiert. Die auskondensierten Brüden werden aufgefangen und vollständig in die Ketonvorlage der Ethinylierung rückgeführt.

Das gereinigte HNER wurde der Trennwandkolonne über einen flüssigen Seitenabzug mit einer Reinheit von 98,5 % entnommen. Im Sumpf der Kolonne wurden Temperaturen von 180°C bis 190°C erreicht. Bei diesen Temperaturen waren nur noch geringe Mengen HNER im Sumpf enthalten. Der Sumpf wurde zur Abtrennung dieses HNER-Gehaltes in einen bei 10 mbar betriebenen Dünnschichtverdampfer gegeben und dessen Kopfprodukt in die Kolonne zurückgefahren. Der Sumpf des Dünnschichtverdampfers wurde aus dem Verfahren ausgeschleust.

Die gewogene, destillierte Gesamtausbeute an HNER über die Stufen a) bis c) betrug 91,9 %, bei einer Selektivität bezogen auf HGAC von 92,0 %. Dieses zeigt in Verbindung mit den Ausführungen zu den Beispielen, dass die Gewinnung des Allylalkohols aus dem entsprechenden Keton nach dem erfindungsgemäßen Verfahren der Stufen a) bis c) auf technisch einfache Weise mit hohen Selektivitäten und Raum-Zeit-Ausbeuten möglich ist.

Das so gewonnene H-Nerolidol kann dann gegebenenfalls in einer kontinuierlichen Carroll-Reaktion weiter zum H-Farnesylaceton umgesetzt werden.

Diese Umsetzung eines Allylalkohols mit einem Acetessigsäureester wird im folgenden Beispiel exemplarisch an der Umsetzung von H-Linalool zum H-Geranylaceton ausgeführt.

### Stufe (d) Herstellung von H-GAC mittels kontinuierlicher Carroll-Reaktion

Als Apparatur wird eine Fraktionierkolonne mit 30 Glockenböden (ca. 20 theoretische Stufen) und einem Innendurchmesser von 30 mm verwendet. Die Numerierung der Böden erfolgt von unten nach oben, d.h. der unterste Boden ist Boden 1 und der oberste Boden ist Boden 30. Die Kolonne ist in regelmäßigen Abständen mit Thermoelementen bestückt, so daß außer am Sumpf und am Kopf der Kolonne an jeder 3. bis 4. theoretischen Stufe die Temperatur gemessen werden kann. Zusätzlich zum Temperaturprofil kann mit Hilfe entsprechender Probennahmestellen das Konzentrationsprofil in der Kolonne ermittelt werden. Der Verdampfer, der mit Hilfe eines Thermostaten auf 250°C beheizt werden kann, hat ein Volumen von ca. 350 ml, wobei der Füllstand während des Betriebs etwa 225 ml beträgt. Auf die Kolonne ist ein Kühler aufgesetzt, der mit einem Kryostaten betrieben wird. Weiterhin ist die Kolonne mit einem Vakuumaggregat und einer Kühlfalle ausgestattet. Alle zu- und ablaufenden Ströme werden mit Hilfe von Waagen erfaßt und aufgezeichnet.

Auf den Boden 27 der Kolonne wurden 135,0 g/h (0,81 mol/h) 3,7-Dimethyl-1-octen-3-ol (Dihydrolinalool) und auf den Boden 3 der Kolonne 94,0 g/h (0,81 mol/h) Acetessigsäuremethylester dosiert. Als Katalysator wurde ein durch Umsetzung von Aluminiumsek.-butylat mit Acetessigsäuremethylester hergestelltes, gemischtes Aluminiumtriacetoacetat als methanolische Lösung eingesetzt. Der Katalysator wurde durch Elementaranalyse und ¹H-NMR charakterisiert, wobei sich ein Aluminiumgehalt 5,0 Gew.-% und ein Umesterungsgrad (Methanol gegen 2-Butanol) von 50 % ergab. 7,6 g/h (1,5 Mol-% Aluminium bezogen Acetessigsäuremethylester) dieses Katalysators wurde zusammen mit dem 3,7-Dimethyl-1-octen-3-ol auf den Boden 27 dosiert. Es wurde ein Systemdruck von 500 mbar und ein Rücklaufverhältnis von 3 eingestellt. Die Sumpftemperatur betrug 200°C und die Verweilzeit in dem Reaktorsystem 2 Stunden. Als Sumpfstrom wurden 171,7 g/h Rohprodukt mit 87,4 Gew.-% 6,10-Dimethyl-undec-5-en-2-on, 3,8 Gew% 3,7-Dimethyl-1-octen-3-ol, 0,4 Gew.-% Acetessigsäuremethylester und 8,4 Gew.-% Hochsieder gewonnen. Am Kopf der Kolonne wurden 23,3 g/h Destillat bestehend aus 89,5 Gew.-% Methanol abgezogen. Das während der Reaktion entstehende Kohlendioxid wurde über Kopf abgeleitet. Es wurde 6,10-Dimethyl-undec-5-en-2-on mit einer Selektivität von 99,2 % bezogen 3,7-Dimethyl-1-octen-3-ol und 95,4 % bezogen Acetessigsäuremethylester erhalten. Der Umsatz betrug 95 % bezogen 3,7-Dimethyl-1-octen-3-ol und 99,2 Gew.-% bezogen Acetessigsäuremethylester. Aus dem Rohprodukt wurde in einer Destillationskolonne mit 22 theoretischen Stufen 6,10-Dimethyl-undec-5-en-2-on in einer Reinheit von 99,99 % erhalten.

Das Beispiel zeigt, dass die Carroll-Reaktion nach dem erfindungsgemäßen Verfahren mit hoher Raum-Zeit-Ausbeute und hoher Selektivität durchgeführt werden kann.

Das in Stufe d) gewonnene Keton kann anschließend wieder in die Stufe a) eingesetzt werden (Stufe e)).

## Patentansprüche

1. Verfahren zur Herstellung von höheren, α,β-ungesättigten Alkoholen der allgemeinen Formel Ia bzw. Ib 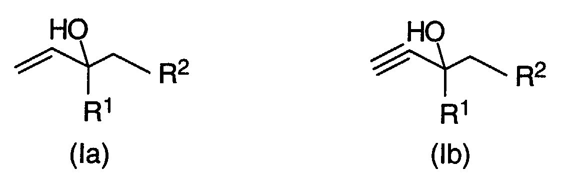 wobei
R¹ Wasserstoff oder einen C₁-C₄-Alkylrest bedeutet
R² die Gruppe der allgemeinen Formel II 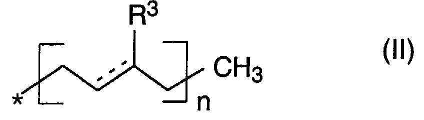 Wasserstoff einen gesättigten oder einen ein- oder mehrfach ungesättigten C₁-C₃₀-Alkyl-, Cycloalkylalkyl-oder Cycloalkylrest bedeuten, die gegebenenfalls mit C₁-C₄-Alkyl substituiert sein können
R³ Wasserstoff oder einen C₁-C₄-Alkylrest bedeutet,
und die gestrichelte Linie eine zusätzliche Doppelbindung bedeuten kann, durch
n die Zahl 0 oder 1 bis 6 bedeutet, durch
a) basekatalysierte Monoethinylierung eines Ketons der allgemeinen Formel R¹-CO-CH₂-R² in flüssigem Ammoniak (NH₃/KOH-Verfahren, bzw. NH₃/MOR-Verfahren), wobei R¹ und R² die oben angegebene Bedeutung besitzen,
b) gegebenenfalls anschließender Hydrierung des Acetylenalkohols der allgemeinen Formel Ib in Gegenwart von Wasserstoff an einem Pd-haltigen Dünnschichtkatalysator und
c) anschließender Reindestillation des Hydrieraustrages, sowie gegebenenfalls
d) Umsetzen des in den Stufen a) bis c) oder a) und c) erzeugten Alkohols der allgemeinen Formel Ia bzw. Ib 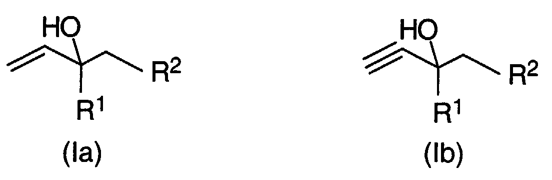 mit Diketen oder Acetessigsäurealkylestern der allgemeinen Formel IV 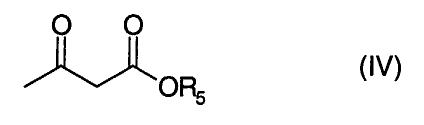 in der R⁵ für C₁-C₄-Alkyl steht,
in Gegenwart von 0,1 bis 0,5 Mol-% einer organischen Aluminiumverbindung bezogen auf den umzusetzenden Acetessigsäurealkylester zur Herstellung des korrespondierenden um 3 C-Atome verlängerten Methylketons,
e) und Einsatz des gegebenenfalls in Stufe d) gewonnenen Ketons in die Stufen a) bis c),
**dadurch gekennzeichnet, dass** in Stufe a) nur ein Teilumsatz des Ketons erzielt wird und das nicht umgesetzte Keton in Stufe c) abgetrennt und zur Stufe a) zurückgeführt wird.

2. Verfahren gemäß Anspruch 1, **dadurch gekennzeichnet, daß** der Teilumsatz des Ketons bei 50 bis 95 % liegt.

3. Verfahren zur Herstellung von höheren Alkoholen der allgemeinen Formel Ia bzw. Ib, gemäß einem der Ansprüche 1 oder 2, **dadurch gekennzeichnet, daß** R₂ die Gruppe der allgemeinen Formel II bedeutet.

4. Verfahren gemäß einem der Ansprüche 1 bis 3, **dadurch gekennzeichnet, daß** im Anschluß an die Umsetzung der Stufen a) bis c) eine Umsetzung gemäß den Stufen d) und e) stattfindet.

5. Verfahren gemäß Anspruch 4, **dadurch gekennzeichnet, daß** die Sequenz der Stufen a) bis d) mehrfach durchlaufen werden kann.

6. Verfahren gemäß einem der Ansprüche 1 bis 5, **dadurch gekennzeichnet, daß** ein entsprechender, um eine Penten-2-on-5-yliden-Gruppe erweiterter Alkohol der allgemeinen Formel Ia bzw. Ib, in Abfolge der Schritte d), und e) erhalten wird.

7. Verfahren gemäß einem der Ansprüche 1 bis 6, **dadurch gekennzeichnet, daß** der in Stufe a) gewonnene Acetylenalkohol der allgemeinen Formel III unter Umgehung der Stufe b) direkt der Reindestillation der Stufe c) unterworfen wird.

8. Verfahren gemäß Anspruch 7, **dadurch gekennzeichnet, daß** im Anschluß an die Umsetzung der Stufen a) und c) eine Umsetzung gemäß den Stufen d) und e) stattfindet.

9. Verfahren gemäß einem der Ansprüche 1 bis 6, **dadurch gekennzeichnet, daß** die höheren Alkohole der allgemeinen Formel Ia, ausgewählt sind aus der Gruppe 3,7,11-Trimethyl-1,6,10-dodekatrien-3-ol (2-NER), 3,7,11-Trimethyl-1,6-dodekadien-3-ol (HNER), 3,7,11-Trimethyldodec-1-en-3-ol (THNER), 3,7,11,15- Tetramethyl-1-hexadecen-3-ol (IP), 3,7-Dimethylocta-1,6-dien- 3-ol (2-LIN), 3,7-Dimethyloct-1-en-3-ol (HLIN), 3-Methyl-1- (2,6,6-trimethyl-1-cyclohexen-1-yl)-penta-1,4-dien-3-ol, 3-Methyl-3-hydroxybuten (MBE).

10. Verfahren gemäß einem der Ansprüche 1 bis 8, **dadurch gekennzeichnet, daß** die Acetylenalkohole der allgemeinen Formel Ib, ausgewählt sind aus der Gruppe 3,7,11-Trimethyldodec-1-yn-3-ol (TMD) 3,7,11,15-Tetramethyl-hexadec-1-yn-3-ol (DIP3,7,11-Trimethyl-6,10-dodekadien-1-yn-3-ol (2-DHNER), 3,7,11-Trimethyl-6-dodecen-1-yn-3-ol (DHNER), 3,7-Dimethylocta-6-en-1-yn-3-ol (2-DHL), 3,7-Dimethyloct-1-yn-3-ol (HDHL), 3-Methyl-1-(2,6,6-trimethyl-1-cyclohexen-1-yl)-penta-4-en-1-yn-3-ol, 3-Methyl-3-hydroxybutin (MBI).

11. Verfahren gemäß einem der Ansprüche 1 bis 10, **dadurch gekennzeichnet, dass** die in Stufe (a) eingesetzten Ketone der allgemeinen Formel R1-CO-R2 sowohl als Reinsubstanzen als auch gemischt miteinander in die Stufe (a) eingesetzt werden können.

12. Verfahren nach einem der Ansprüche 1 bis 11, **dadurch gekennzeichnet, dass** die Ethinylierung in einem Rohrreaktor mit einer Kesselzahl größer 50 durchgeführt wird.

13. Verfahren nach einem der Ansprüche 1 bis 12, **dadurch gekennzeichnet, dass** das in Stufe (a) nach einer Teilstrecke des Reaktors umgesetzte Acetylen durch frisches Acetylen oder durch frisches Acetylen gelöst in Ammoniak nach dieser Teilstrecke ersetzt wird.

14. Verfahren nach einem der Ansprüche 1 bis 13, **dadurch gekennzeichnet, dass** in Stufe (a) Kaliummethanolat in Methanol als Katalysator verwendet wird.

15. Verfahren gemäß einem der Ansprüche 1 bis 14, **dadurch gekennzeichnet, dass** die Acetylenalkohole der allgemeinen Formel Ib sowohl als Reinsubstanzen als auch gemischt miteinander in der Hydrierung umgesetzt werden können.

16. Verfahren gemäß einem der Ansprüche 1 bis 15, **dadurch gekennzeichnet, dass** vor der destillativen Auftrennung des Reaktionsaustrages aus Stufe a) und b) eine Kurzwegverdampfung zur schonenden Abtrennung hochsiedender Nebenprodukte durchgeführt wird.

17. Verfahren gemäß einem der Ansprüche 1 bis 16, **dadurch gekennzeichnet, daß** zur destillativen Auftrennung des Reaktionsaustrages aus Stufe a), b) und d) thermisch gekoppelte Kolonnen verwendet werden.

18. Verfahren gemäß Anspruch 17, **dadurch gekennzeichnet, daß** als Kolonnen Trennwandkolonnen verwendet werden.

19. Verfahren nach Anspruch 18, **dadurch gekennzeichnet, daß** die Trennwandkolonne mit mehr als einem Seitenabzug ausgestattet ist.

20. Verfahren gemäß einem der Ansprüche 1 bis 19, **dadurch gekennzeichnet, daß** in die Ethinylierung der Stufe (a) Ketone eingesetzt werden, die durch partielle oder vollständige selektive C=C-Hydrierung von 6,10-Dimethylundeca-3,5,9-trien-2-on (Pseudoionon) oder 6,10,14-Trimethylpenta-5,9,13-trien-2-on erhalten werden.
